# EUROPÄISCHE PATENTSCHRIFT

(11) **EP 1 972 259 B1**
(45) Veröffentlichungstag und Bekanntmachung des Hinweises auf die Patenterteilung: **28.11.2012**
(21) Anmeldenummer: 08005256.6
(22) Anmeldetag: 20.03.2008
(51) Int. Cl.: A61B 1/005, A61B 1/01

(54) **Ablenkbares autoklavierbares Endoskop**
Deflectable autoclaving endoscope
Endoscope autoclavable pouvant être dévié

(30) Priorität: 20.03.2007 DE 102007014739
(43) Veröffentlichungstag der Anmeldung: 24.09.2008
(73) Patentinhaber: Karl Storz GmbH & Co. KG, 78532 Tuttlingen (DE)
(72) Erfinder: Irion, Klaus M., Dr.-Ing., 78576 Emmingen-Liptingen (DE); Rüegg, Thomas, Vancouver BC, V5Z 4K6 (CA)
(74) Vertreter: Weller, Wolfgang

(56) Entgegenhaltungen:
- DE-A1- 4 222 271
- DE-A1- 4 442 185
- DE-C1- 19 955 229
- US-A- 5 976 075

## Beschreibung

Die Erfindung betrifft ein Endoskop mit einem ein Beleuchtungs- und Bildübertragungssystem aufnehmenden Schaft, wobei an den Schaft ein Außenteil angelegt ist, das einen Ablenkmechanismus zum Ablenken des Endoskopes aufweist, wobei das Außenteil zum Verbinden mit dem Schaft seitlich an diesen anlegbar ist.

Eine derartige Kombination aus einem Endoskop und einem daran angelegten Außenteil ist aus der US-A-5 976 075 bekannt.

Das Endoskop als solches ist als relativ kurzer Stummel ausgebildet von dem sich proximalseitig ein flexibles Kabel weg erstreckt. Das Außenteil ist seitlich an den Endoskopkörper angebracht, das flexible Kabel des Endoskopes erstreckt sich durch das Außenteil hindurch. Das Außenteil weist einen Ablenkmechanismus auf, bei dessen Verschwenken das gesamte Endoskop mitverschwenkt wird.

Derartige Endoskope sind weit verbreitet und werden in der minimal-invasiven Chirurgie eingesetzt. Bei starren Endoskopes besteht der Schaft aus einem steifen Material, meist Medizinerstahl. Bei flexiblen Endoskopen besteht der Schaft zumindest im flexiblen Bereich aus einem biegsamen Kunststoffmaterial.

Der Schaft des Endoskops kommt bei der Anwendung unmittelbar in Berührung mit dem Gewebe und ist somit Kontaminationen ausgesetzt. Aus ökonomischen Gründen besteht das Interesse, solche Endoskope vielfach verwenden zu können.

Dazu müssen die Endoskope nach einer Anwendung zunächst einmal gereinigt werden und anschließend sterilisiert werden, wobei dies meistens über einen Autoklaviervorgang durchgeführt wird.

Bei dem Autoklavieren wird das Endoskop relativ hohen Temperaturen ausgesetzt, beim sogenannten Blitzautoklavieren Temperaturen von über 134° bis zu 165°C. Zusätzlich werden manchmal im Autoklav noch Chemikalien, beispielsweise peroxidhaltige Chemikalien hinzugefügt, um eine absolute Sterilität zu erzielen.

Da die minimalinvasive Chirurgie immer weiter verbreitet ist, finden diese Zyklen mehrfach täglich statt, so dass solche Endoskope erheblichen Belastungen ausgesetzt sind, denen diese auf Dauer widerstehen müssen.

Bei starren Endoskopen ist das durch entsprechende metallische Materialien zu erzielen.

Bei flexiblen Endoskopen ist dies nicht mit hoher Sicherheit zu erzielen, da die flexiblen Materialien, meist Kunststoffmaterialien, diesen harten Bedingungen auf Dauer nicht widerstehen. Die Flexibilität eröffnet allerdings der Handhabungsperson wesentlich größere Blickbereiche, nämlich indem das distale Ende gekrümmt werden kann, so dass nicht nur ein Geradeausblick, sondern auch ein seitlicher Rundumblick eröffnet wird.

Bei starren Endoskopen behilft man sich dadurch, dass das distale Ende durch entsprechend abgeschrägte Objektive nicht nur einen Geradeausblick (0°-Blick) sondern auch abgewinkelte Schrägblicke bis zu 45° erlaubt.

Es besteht aber ein Bedarf an autoklavierbaren Endoskopen, die eine flexible Bildrichtung ermöglichen.

Aus der US 5,402,768 ist bekannt geworden, den Schaft eines Endoskopes so auszubilden, dass ein innerer Schaftkern die optischen Elemente des Bildübertragungssystems aufnimmt. Dies können klassischerweise starre Glaslinsen sein, oder bei moderneren Konstruktionen Bildaufnahmeelemente in Form von Halbleiterbildsensoren (CCD, CMOS). Das einfallende Bild wird über ein Objektiv auf den Halbleiterbildsensor abgebildet und dort in elektrische Signale umgewandelt, die über Kabel im Inneren des Schaftes zum proximalen Ende des Endoskops geleitet werden. Diese Signale werden dann verarbeitet und meist auf einem Monitor als Bild dargestellt. Da die Baueinheiten eines Halbleiterbildsensors immer kleiner werden, können diese insbesondere bei flexiblen Endoskopen eingesetzt werden, da eine Krümmung des Schaftes diese kleinen Bauteile nicht beeinträchtigt.

Allerdings stellt sich hier das Problem der Autoklavierbarkeit.

Bei der US 5,402,768 wird dem dadurch abgeholfen, dass über den inneren Kern des Schaftes ein diesen komplett abdeckender am distalen Ende hermetisch abgeschlossener schlauchartiger Überzug aufgeschoben wird. Am proximalen Ende weist der Schlauch sogar noch faltenbalgartige Aufstauchungen auf, die ebenfalls zum Schutz über den gesamten proximalen Endbereich des Schaftes, somit auch des Endoskops übergezogen werden können.

Der Schaft selbst kommt daher bei einem Eingriff nicht mehr mit dem Gewebe in Berührung, sondern nur die äußere übergeschobene Hülle.

Nach einem Einsatz wird die Hülle wieder abgezogen und verworfen.

In einer Ausgestaltung sind in der äußeren Umhüllung Steuerdrähte integriert, über die der Zusammenbau aus flexiblem Schaft und äußerer flexibler Umhüllung gekrümmt werden kann.

Dieses Endoskop ist umständlich zu handhaben, denn vor jedem Gebrauch muss auf den langen dünnen Schaft des Endoskops der sterile Schlauch aufgeschoben werden, was einer gewissen Geschicklichkeit bedarf. Es kann nicht ausgeschlossen werden, dass bei diesen vorbereitenden Handhabungen Kontaminationen an den Schaft gelangen.

Wesentlich größer ist die Kontaminationsgefahr, wenn nach dem Einsatz der Schlauch von dem Schaft des Endoskops wieder abgenommen und verworfen wird. Daher ist es notwendig, den inneren Kern, der das Bildübertragungssystem enthält zumindest durch Desinfektionsflüssigkeiten zu sterilisieren. Diese Vorgänge sind aber sehr langwierig und nach zahlreichen Einsätzen kann nicht ausgeschlossen werden, dass die aggressiven chemischen Stoffe die aus Kunststoff bestehende Wand des Kerns durchdringen und in das Innere des Kerns eintreten.

Aus der DE 44 42 185 A1 ist eine Führung insbesondere für ein flexibles Endoskop mit einem rohrförmigen abwinkelbaren Schaft bekannt. Der Schaft weist mindestens einen flexiblen Abschnitt auf und es sind Steuermittel vorgesehen, mit denen das distale Schaftende durch Betätigung vom proximalen Ende her gegenüber dem proximalen Schaftteil abwinkelbar ist.

Es ist Aufgabe der vorliegenden Erfindung ein autoklavierbares Endoskop zu schaffen, das ablenkbar ist und das einfach zu handhaben ist.

Erfindungsgemäß wird die Aufgabe dadurch gelöst, dass im flexiblen Bereich am Schaft ein Rückstellelement vorhanden ist, das kein Bauteil des Außenteils ist, durch das Schaft und starrer distaler Endabschnitt in eine geradlinige Ausrichtung verbleiben, und dass der Schaft proximalseitig des flexiblen Bereiches in dem Abschnitt, an dem das Außenteil angelegt ist, starr ausgebildet ist, und dass der Schaft aus metallischem Material hergestellt ist und wie bei einem starren metallischen Endoskop autoklavierbar ist.

Diese Maßnahme haben nun mehrere Vorteile.

Die empfindlichen Bauelemente wie Bildübertragungssystem und auch Beleuchtungssystem können hermetisch abgeschlossen im Schaft aufgenommen werde.

Zum Ablenken wird ein separates Teil, nämlich das Außenteil angelegt, das den Ablenkmechanismus aufweist. Da das Außenteil seitlich an den Schaft angelegt werden kann oder umgekehrt der Schaft seitlich in das Außenteil eingelegt werden kann, ist diese Handhabung einfach. Dies kann beispielsweise durch ein einfaches Einlegen, Einclipsen oder Einschnappen erfolgen. Durch Vorsehen eines distal ablenkbaren Endabschnittes des Schaftes muss nur ein bestimmter Bereich des Schaftes eine gewisse Flexibilität aufweisen, um diese Ablenkung zu ermöglichen. Dies ist auch mit relativ steifen oder metallischen Materialien zu erzielen, beispielsweise durch faltenbalgartige Ausgestaltungen oder Materialverdünnungen, so dass der Schaft nicht insgesamt aus einem flexiblen Material hergestellt werden muss. In anderen Worten ausgedrückt, kann der Schaft nach wie vor komplett aus metallischem Material hergestellt sein, er muss nur die Flexibilität an der Ablenkstelle im distalen Bereich zeigen. Das Außenteil enthält den Ablenkmechanismus, über den, nach seitlichem Anlegen von Außenteil an Schaft oder umgekehrt, der distale Endabschnitt des Schaftes abgelenkt werden kann.

Ein weiterer Vorteil besteht darin, dass ohne das Außenteil das Endoskop als ein "klassisches" starres Endoskop eingesetzt werden kann, d.h. dass dann das Außenteil gar nicht angelegt wird, sondern nur im Bedarfsfalle, wenn eine Ablenkung gewünscht wird. Dies ist einfach und rasch durchzuführen indem nämlich das Außenteil einfach seitlich an den Schaft angelegt wird.

Dies kann auch im Laufe einer Operation erfolgen und muss nicht zwingend vor einer Operation erfolgen, so dass nicht andauernd das Außenteil am Schaft vorhanden sein muss.

Nach dem Eingriff kann das Außenteil vom Schaft einfach wieder abgenommen werden und je nach Ausbildung separat gereinigt und sterilisiert werden oder, wenn es als Wegwerfteil ausgebildet wird, schlicht und einfach entsorgt werden.

Das Endoskop selbst, ohne Außenteil, kann den üblichen Autoklaviervorgängen unterworfen werden. Es ist somit keine Vorsorge wie beim eingangs genannten Stand der Technik zu treffen, wonach der Schaft vollkommen durch das Außenteil abgedeckt ist. Dadurch ist eröffnet, das Außenteil entsprechend mit seitlichen Öffnungen oder Schlitzen zu versehen, über die es in einem einfachen seitlichen Ansetzvorgang an den Schaft angebracht werden kann.

Das Bildübertragungs- und Beleuchtungssystem ist von dem Ablenkmechanismus vollständig getrennt, so dass keine dichtungskritischen Verbindungen oder Durchführungen zwischen diesen Bauelementen vorhanden oder notwendig sind.

Bei der Erfindung weist der Schaft im Übergangsbereich von ablenkbarem Endabschnitt zum Schaft einen flexiblen Bereich auf.

Diese Maßnahme hat, wie zuvor schon angedeutet, den Vorteil, dass dieser flexible Bereich nur in dem Bereich vorhanden sein muss, in dem die Ablenkung bewerkstelligt werden soll.

Bei der Erfindung ist im flexiblen Bereich ein Rückstellelement vorhanden, durch das der Schaft und der distale Endabschnitt in eine lineare Ausrichtung bringbar ist.

Diese Maßnahme hat den handhabungstechnisch erheblichen Vorteil, dass, falls keine Ablenkung erwünscht ist, der Ablenkmechanismus schlicht und einfach freigegeben wird und das Rückstellelement diesen abgelenkten Bereich wieder in die geradlinige Ausrichtung zurückbringt.

Diese Maßnahme hat auch den Vorteil, dass, falls das Endoskop ohne das Außenteil eingesetzt wird, sichergestellt ist, dass durch das Rückstellelement das Endoskop in der geradlinigen Ausrichtung verbleibt. Das Rückstellelement stellt also sicher, dass trotz der flexiblen Ablenkstelle der Schaftkörper insgesamt ein steifer, starrer Körper ist, wenn nicht die Ablenkkraft des Ablenkmechanismus auf ihn einwirkt. Das erhöht erheblich die Flexibilität und erleichtert auch die Handhabbarkeit des Endoskops als starres Endoskop.

Bei der Erfindung ist der Schaft proximalseitig des flexiblen Bereiches starr ausgebildet.

Zusätzlich dazu ist der distale Endabschnitt starr ausgebildet sein.

Diese Maßnahmen haben den Vorteil, dass abseits der Ablenkstelle der Schaft wie bei einem starren Endoskop ausgebildet ist, also den entsprechenden mechanischen Schutz, der in diesen aufgenommenen Beleuchtungs- und Bildübertragungssystemen sicherstellt und auch die Handhabung des Endoskops wie ein starres Endoskop ermöglicht. Starre Endoskope werden in der minimalinvasiven Chirurgie ja üblicherweise über Trokare in den Körper eingeführt, was nun mit dem erfindungsgemäßen Endoskop trotz seiner Ablenkbarkeit nach wie vor möglich ist, insbesondere auch dann, wenn kein Außenteil angelegt ist.

In einer Ausgestaltung der Erfindung ist das Rückstellelement als federndes Element ausgebildet.

Diese Maßnahme hat den Vorteil, dass die Rückstellkraft durch ein solches federndes Element bereitgestellt wird, wobei solche Elemente aus robusten, insbesondere metallischen Materialien herstellbar sind, die den harten Bedingungen widerstehen. So kann beispielsweise das federnde Element eine Schraubenlinienfeder sein, die um die Außenseite des Schaftes gelegt ist und zwar in dessen flexiblen Bereich. Dadurch wird nicht nur die Rückstellkraft bei der Ablenkung bewerkstelligt, sondern die Feder stellt zugleich einen mechanischen Schutz für den flexiblen Bereich dar. Wenn beispielsweise der flexible Bereich des Schaftes durch Materialschwächungen der Schaftwand geschaffen wurde, trägt die darum gewundene Feder zur mechanischen Stabilität dieses Bereiches bei.

In einer weiteren Ausgestaltung der Erfindung weist der distale Endabschnitt gegenüber dem Schaft einen größeren Durchmesser auf.

Ein beachtenswerter Vorteil besteht darin, dass durch den durchmessergrößeren Bereich mehr Kraftansatzpunkte für den Ablenkmechanismus bestehen, um diesen Bereich aus der geradlinigen Richtung abzulenken. Das Außenteil, das den Schaft in diesem ablenkbaren und distalen Endabschnitt umgreift, ist dann entsprechend mit einem größeren Durchmesser versehen, so dass genügend Ansatzpunkte außerhalb der Symmetrieachse vorhanden sind, um beispielsweise durch eine Zugkraft abseits der Symmetrieachse eine Auslenkung zu bewirken. Ein weiterer Vorteil besteht darin, dass man den distalen Endabschnitt mit größerem Durchmesser vollständig durch den Trokar hindurchschieben kann, so dass danach neben dem noch im Trokar befindlichen Schaftabschnitt mit geringerem Durchmesser ausreichend Raum zur Verfügung steht, um weitere Instrumente durch den Trokar hindurchzuführen.

In einer weiteren Ausgestaltung der Erfindung weist der Schaft proximalseitig einen durchmessergrößeren Abschnitt auf.

Diese Maßnahme hat den Vorteil, dass über diesen durchmessergrößeren proximalen Endbereich die Handhabung erleichtert ist, dieser quasi als eine Art Handgriff fungiert, mit dem der Zusammenbau aus Schaft und Außenteil gehandhabt werden kann. Dies erleichtert auch das Anlegen oder Abnehmen der beiden Teile, insbesondere, wenn es sich um ein dünnes Endoskop handelt.

In einer weiteren Ausgestaltung der Erfindung ist das Außenteil als ein zumindest teilweise seitlich offenes, schalenartiges Element ausgebildet.

Diese Maßnahme hat den Vorteil, dass das schalenartige Element durch eine einfache seitliche Anlegebewegung, gegebenenfalls kombiniert noch mit einer anschließenden axialen Verschiebung, an den Schaft angelegt werden kann. Zugleich ist ein solches schalenartiges Element ausreichend stabil, um den Ablenkmechanismus zu tragen und auch die Ablenkkräfte bzw. die Widerstandskräfte des ablenkbaren distalen Endabschnittes des Schaftes aufzunehmen bzw. zu überwinden.

In einer weiteren Ausgestaltung der Erfindung ist die seitliche Öffnung als Langschlitz ausgebildet, über die der Schaft seitlich einclipsbar ist.

Diese Maßnahme hat den Vorteil, dass dieser Langschlitz ein Orientierungsmerkmal darstellt, die es der Handhabungsperson erleichtert den Zusammenbau von Schaft und Außenteil zu bewerkstelligen.

In einer weiteren Ausgestaltung der Erfindung sind proximalseitig und distalseitig am Außenteil durchmessererweiterte Bereiche vorhanden, in die die durchmessergrößeren Bereiche des Schaftes formschlüssig einbringbar sind.

Diese Maßnahmen haben den Vorteil, dass der Zusammenbau aus Schaft und Außenteil insbesondere in diesen proximalseitigen und distalseitigen Stellen sehr intensiv und großflächig ist.

In einer weiteren Ausgestaltung der Erfindung sind die durchmessererweiterten Bereiche des Schaftes axial in die durchmessererweiterten Bereiche des Außenteils einschiebbar.

Diese Maßnahme hat den Vorteil, dass das Außenteil zunächst in etwas axial verschobenem Zustand seitlich angelegt werden kann und dann durch eine axiale Relativverschiebung die durchmessergrößeren Bereiche des Schaftes in die entsprechend durchmessererweiterten Abstände des Außenteiles axial eindringen. Der seitliche Schlitz kann so bemessen werden, dass die durchmessergrößeren Bereiche des Schaftes nicht seitlich über den Schlitz in diesen durchmessererweiterten Bereichen des Außenteils heraustreten können. Das stellt dann somit zugleich eine Abfallsicherung dar.

Wie bereits erwähnt, werden Endoskope bei der minimalinvasiven Chirurgie über Trokare in den Körper, z.B. in das Abdomen eingeführt. Zur Vergrößerung der inneren Hohlräume, werden diese mit einem Gas, meist CO₂, aufgebläht. Daher muss ein Endoskop zur Vermeidung von Gasentweichungen möglichst gasdicht durch den Trokar in die Körperhöhle eingeführt werden.

Die durchmessererweiterten Bereiche können nur so gewählt werden, dass deren Außendurchmesser etwa dem lichten Innendurchmesser des Trokars entsprechen, wodurch ein gasdichter Abschluss erzielt werden kann.

Wird das Endoskop eingesetzt, entspricht zumindest dessen proximalseitiger erweiterter Bereich dem Trokar. Wenn der Zusammenbau aus Schaft und Außenteil eingeführt wird, entspricht der erweiterte Bereich des Außenteils dem Trokar.

In einer weiteren Ausgestaltung der Erfindung ist eine Mittellängsachse des distalen Endabschnittes des Außenteils seitlich versetzt zur Mittellängsachse eines mittigen Abschnittes des Außenteils.

Es ist ja bereits angesprochen, dass der Zusammenbau aus Endoskop und Außenteil wie ein starres Endoskop durch ein Trokar hindurch in den Körper eingebracht werden kann. Die seitliche Bewegungsfreiheit des Ablenkmechanismus ist dann zumindest in dem Bereich, in dem das Endoskop im Trokar aufgenommen ist durch den lichten Innendurchmesser des Trokars begrenzt. Die Auslenkung des distalen Endabschnittes findet selbstverständlich erst statt, wenn dieser durch den Trokar hindurch in die Körperhöhle eingeschoben ist, dennoch verbleibt ein relativ langer axialer Abschnitt des Endoskops im Inneren des Trokars, der die seitlichen Bewegungen begrenzt.

Diese asymmetrische bzw. nicht koaxiale Anordnung erlaubt abseits der Symmetriemittellängsachse liegende Kraftansetzpunkte zu realisieren, um die Auslenkung zu bewirken.

In einer weiteren Ausgestaltung der Erfindung ist der seitliche Versatz derart, dass mittiger Abschnitt und distaler Endabschnitt des Außenteils zumindest eine gemeinsame Mantellinie aufweisen.

Diese Maßnahme hat den erheblichen Vorteil, dass ein relativ großer seitlicher Versatz möglich ist, somit abseits der Symmetrieachse liegende Ansatzpunkte für den Ablenkmechanismus offen stehen, durch die gemeinsame Mantellinie aber ein exaktes Führen des Außenteils an der Innenwand des Trokars beim Einführen in den Körper gewährleistet ist.

Dies erleichtert ebenfalls erheblich die Handhabung des Endoskops.

In einer Ausgestaltung der Erfindung ist der Ablenkmechanismus derart ausgestaltet, dass durch diesen ein distaler Abschnitt des Außenteils ablenkbar ist, in dem der distal ablenkbare Endabschnitt des Schaftes aufgenommen ist.

Diese Maßnahme hat den Vorteil, dass das Außenteil stabil ausgebildet werden kann, dessen distaler Abschnitt, der den distal passiv ablenkbaren Endabschnitt des Schaftes aufnimmt, über den Mechanismus abgelenkt werden kann. Dies trägt insgesamt zur Stabilität und zur sicheren Handhabbarkeit des Zusammenbaus beim Ablenken bei.

In einer weiteren Ausgestaltung der Erfindung weist der Ablenkmechanismus ein sich von distal nach proximal erstreckendes Betätigungselement auf, das distal mit dem ablenkbaren Abschnitt des Außenteils und proximalseitig mit einem Schwenkhebel verbunden ist.

Diese Maßnahme hat den Vorteil, dass durch das langerstreckende Betätigungselement eine schlanke Bauweise erzielt wird und der Zusammenbau, wie bereits erwähnt, über einen Trokar im Körper eingeführt und gehandhabt werden kann, wobei der proximalseitige Schwenkhebel von der Bedienungsperson einfach zu ergreifen und zu betätigen ist, was die Handhabbarkeit sehr vereinfacht.

In einer weiteren Ausgestaltung ist das Außenteil als Wegwerfteil, z.B. als Kunststoffteil, ausgebildet.

Durch die zuvor erwähnte einfache mechanische Ausgestaltung ist es möglich, das Außenteil als kostengünstiges Wegwerfteil auszubilden, so dass es nicht gereinigt und sterilisiert werden muss.

In einer weiteren Ausgestaltung der Erfindung ist im ablenkbaren distalen Endabschnitt des Schaftes ein Bilderfassungssystem des Bildübertragungssystems aufgenommen.

Diese Maßnahme hat den Vorteil, dass das Endoskop als Videoendoskop ausgebildet werden kann und das Bilderfassungssystem in dem ablenkbaren distalen Endabschnitt sicher aufgenommen und geschützt ist.

In einer weiteren Ausgestaltung der Erfindung weist das Bilderfassungssystem mindestens einen elektronischen Bildsensor mit einem Objektiv auf.

Diese Maßnahme hat den Vorteil, dass über das Objektiv das Bild fokussiert auf den Sensor gebracht werden kann, so dass ein hervorragendes Bild erzeugt werden kann, wobei diese Bauteile geschützt in dem ablenkbaren distalen Endabschnitt des Schaftes aufgenommen sind. Als Bildsensoren sind Miniatur-CCD's oder CMOS-Sensoren mit entsprechender Pixelauflösung geeignet.

In einer weiteren Ausgestaltung der Erfindung ist ein Sensor vorhanden, der über Gravitationswirkung eine Position bzw. die Richtung "oben" erkennbar macht.

Diese Maßnahme hat den Vorteil, dass der Handhabungsperson über diesen Sensor diese Position angezeigt werden kann, wenn er beispielsweise im Innern des Körpers den distalen Endabschnitt ablenkt und das Endoskop um seine Längsachse dreht.

In einer weiteren Ausgestaltung der Erfindung ist im distalen Endabschnitt des Schaftes ein Mikromotor angeordnet, der mit dem Bildsensor gekoppelt ist und diesen um dessen Bildsensorachse dreht.

Diese Maßnahme hat den Vorteil, dass durch das Drehen das Bild wieder in eine bestimmte Ausrichtung, beispielsweise eine stehende Ausrichtung, gebracht werden kann, die dem Operateur die Orientierung erleichtert.

In einer weiteren Ausgestaltung der Erfindung ist der Mikromotor über eine Kontrolleinheit ansteuerbar, die unter Einbeziehung der Information des Positionssensors den Bildsensor derart dreht, dass nach Ablenkung des distalen Endabschnittes die Bildinformation auf einem Monitor in eine jeweils gleiche Ausrichtung bringbar ist.

Diese Maßnahme hat, wie zuvor erwähnt, den Vorteil der Orientierung für die Handhabungsperson, wobei hier der besondere Vorteil besteht, dass dies über die Kontrolleinheit steuerbar und auch automatisierbar ist.

Diese Bauteile können in dem ablenkbaren distalen Endabschnitt mechanisch geschützt und hermetisch von der Außenwelt abgeriegelt aufgenommen werden, so dass über die Ablenkstelle hinaus und durch den Schaft nur noch Kabel zum proximalen Ende geführt werden müssen, die die entsprechenden Impulse, Signale, Licht und dergleichen leiten. Diese Kabel können an der Innenseite des Schaftes im ablenkbaren Bereich des Schaftes zusätzlich dichtend vergossen eingebracht werden, um selbst in dieser kritischen Stelle für eine absolute Dichtheit und auch für eine zusätzlichen mechanischen Schutz zu sorgen.

Dies trägt insbesondere dazu bei, dass ein solches ablenkbares Endoskop häufige Zyklen von Benutzung/Reinigung/Autoklavierung durchlaufen kann und dennoch seine volle sichere Funktionsfähigkeit behält.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert.
Es zeigen:
- Fig. 1: eine perspektivische Darstellung eines erfindungsgemäßen Endoskops, d.h. dessen Schaft an den ein Außenteil angelegt ist,
- Fig. 2: eine der von Fig. 1 vergleichbare perspektivische Explosionsdarstellung, bei dem das Außenteil vom Schaft abgenommen ist,
- Fig. 3: eine Seitenansicht des Endoskops von Fig. 1,
- Fig. 4: eine der Darstellung von Fig. 3 entsprechende Darstellung mit abgelenktem distalen Endabschnitt, wobei hier zusätzlich noch eine Kontrolleinheit und ein Monitor dargestellt sind, die mit dem Endoskop verbunden sind,
- Fig. 5: eine stark vergrößerte Darstellung des Schaftes des Endoskops teilweise im Längsschnitt im distalen Bereich,
- Fig. 6: einen Schnitt längs der Linie VI-VI, und
- Fig. 7: eine der Schnittdarstellung von Fig. 6 vergleichbare Schnittdarstellung, allerdings mit an den Schaft angelegtem Außenteil.

Ein in den Figuren dargestelltes Endoskop ist in seiner Gesamtheit mit der Bezugsziffer 10 bezeichnet.

Das Endoskop 10 weist einen lang erstreckten geradlinigen steifen Schaft 12 auf, der einen distalen Endabschnitt 14 aufweist.

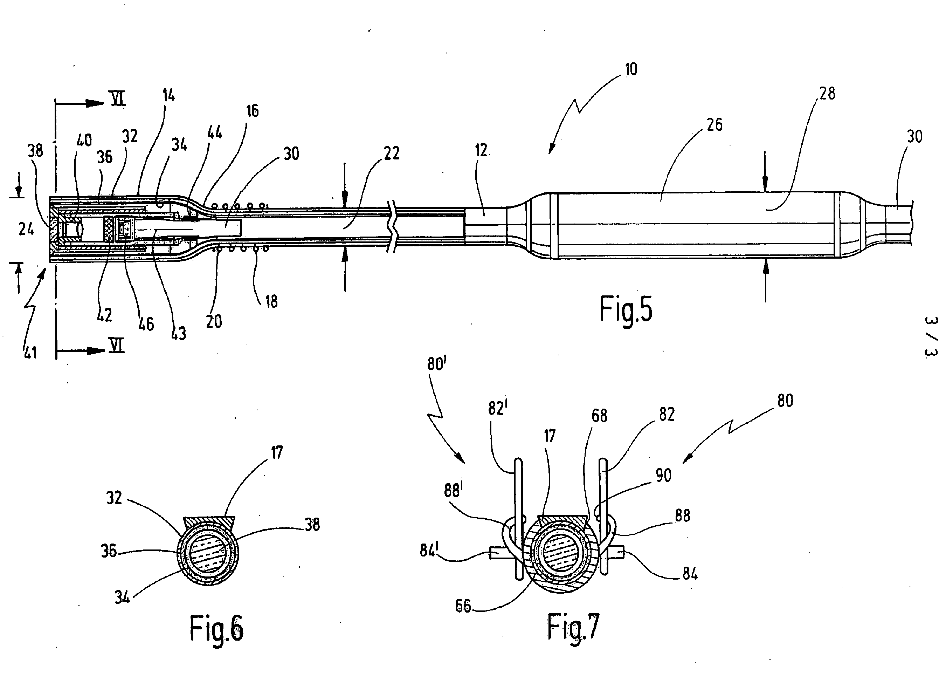

Der Übergang vom Schaft 12 zum distalen Endabschnitt 14 weist einen flexiblen Bereich 16 auf. Dies erfolgt durch eine relative Verdünnung des Materials der Schaftwand in diesem Bereich. In diesem Bereich ist um den Schaft 12 ein Rückstellelement 18 in Form einer schraubenlinienförmigen Feder 20 gelegt. An der Außenseite des distalen Endabschnittes 14 ist ein Ansatz 17 angeformt, der, wie das insbesondere aus Fig. 6 ersichtlich ist, eine etwa schwalbenschwanzförmige Kontur aufweist.

Wie insbesondere aus der Darstellung von Fig. 5 ersichtlich, ist der Außendurchmesser 22 des Schaftes 12 geringer als der Außendurchmesser 24 des distalen Endabschnittes 14.

Wie ebenfalls aus Fig. 5 ersichtlich, weist der Schaft 12 einen proximalen Abschnitt 26 auf, dessen Außendurchmesser 28 ebenfalls größer ist als der Außendurchmesser 22 des Schaftes und der in etwa dem Außendurchmesser 24 des distalen Endabschnittes 14 entspricht. Proximalseitig vom proximalen Abschnitt 26 wird ein Kabel 30 abgeführt, dessen Aufgabe und Funktion später erläutert werden.

Wie insbesondere aus der Schnittdarstellung von Fig. 5 ersichtlich weist der Schaft eine Außenhülle 32 und eine von dieser etwas nach innen beabstandeten Innenhülle 34 auf. In dem ringförmigen Zwischenraum zwischen Außenhülle 32 und Innenhülle 34 sind Glasfasern 36 angeordnet, die Licht von einer (hier nicht dargestellten) Lichtquelle von proximal bis zum distalen Lichtaustrittsende führen, wie das ja im Endoskopbau bei starren Endoskopen üblich ist. Der distale Endabschnitt 14 ist distalseitig mit einer Scheibe 38 hermetisch dicht abgeschlossen. An der Innenseite der Scheibe 38 ist eine sogenannte Optikpatrone 40 angeordnet, also ein Objektiv. Diese stellen somit Bauelemente eines Bilderfassungssystems 41 dar. Ein weiteres Bauteil des Bilderfassungssystems stellt ein Bildsensor 42 dar, der beispielsweise aus einem CCD-Chip besteht, der Licht in elektrische Signale umwandelt.

Der Bildsensor 42 ist mit einem Mikromotor 46 verbunden, der den Bildsensor 42 um dessen Chipachse 43 drehen kann. Ferner ist noch ein Sensor 44 vorhanden, der als Positionssensor ausgebildet ist, der über die Gravitationswirkung beispielsweise die Position bzw. die Richtung "oben" erkennt. Dieser Sensor 44 kann auch an einer anderen Stelle des Schaftes angeordnet sein.

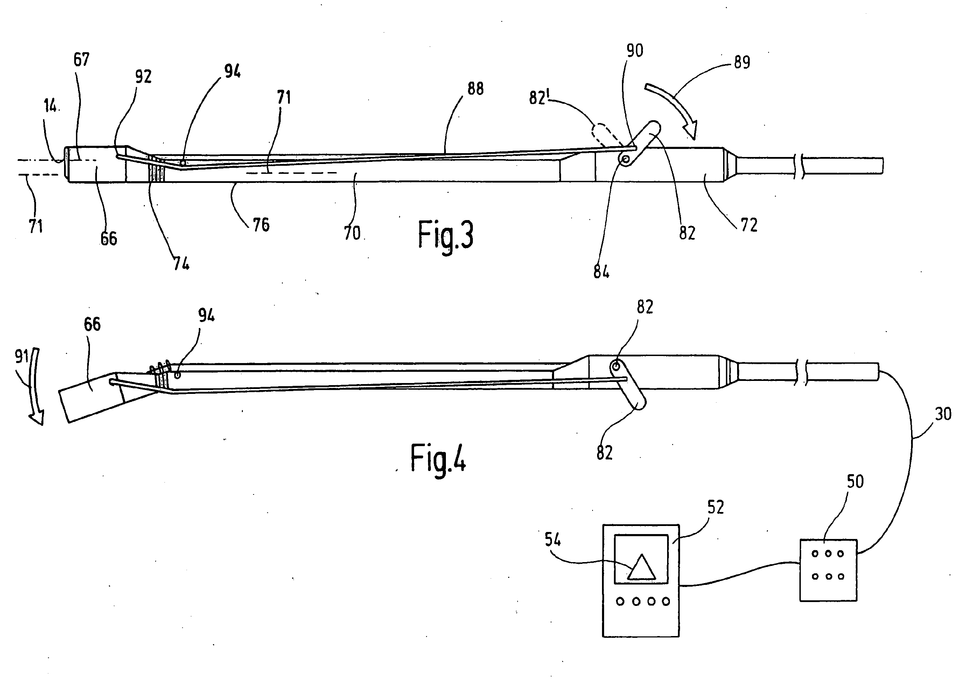

Diese ganzen elektronischen Bauteile sind mit ihren entsprechenden Leitungen zu einem Gesamtkabel 30 zusammengeführt, das im Inneren des Schaftes 12 bis zum proximalen Ende geführt wird und dort, wie das beispielsweise aus Fig. 4 ersichtlich ist, einer Kontrolleinheit 50 zugeführt werden kann, die an einem Monitor 52 ein Bild 54 erzeugt. Diese Bauteile stellen somit insgesamt das Bildübertragungssystem dar, das zum großen Teil im Schaft aufgenommen ist.

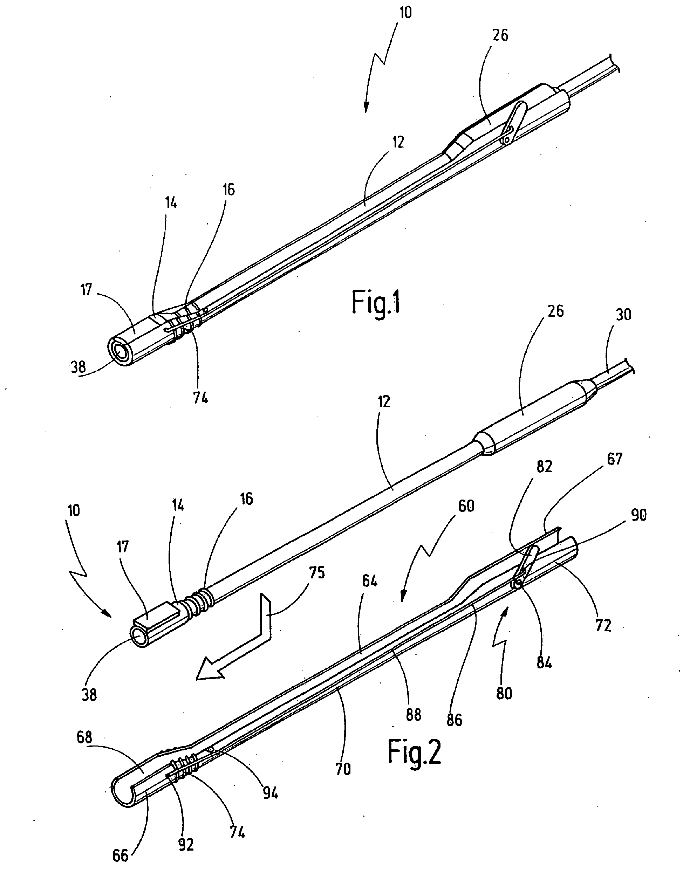

Zurückkehrend zu Fig. 1 und 2 ist ersichtlich, dass an den Schaft 12 ein Außenteil 60 anlegbar ist.

Das Außenteil 60 besteht aus einer langerstreckten Schale 62, die einen seitlichen Schlitz 64 aufweist. Ein distaler Abschnitt 66 der Schale 62 weist im Bereich des Schlitzes eine Öffnung 38 auf, deren Breite so ausgebildet ist, dass gerade der schwalbenschwanzförmige Ansatz 17 des distalen Endabschnittes 14 des Schaftes 12 darin aufgenommen werden kann. Dies ist insbesondere beim Übergang von Fig. 6 zu Fig. 7 bzw. beim Übergang von Fig. 2 zu Fig. 1 ersichtlich.

Die Schale 62 weist ferner einen mittigen Abschnitt 70 auf, der zur Aufnahme des mittigen durchmesserdünneren Bereiches des Schaftes 12 dient.

Dementsprechend weist die Schale 62 einen proximalen Abschnitt 72 auf, der so ausgebildet ist, dass darin der proximale Abschnitt 26 des Schaftes 12 aufgenommen werden kann.

Die Breite des Schlitzes 64 in der Schale 62 kann so ausgebildet werden, dass der Schaft 12 seitlich über den Schlitz 64 gegebenenfalls unter geringer Aufweitung desselben in das Außenteil 60 eingedrückt bzw. eingeclipst werden kann.

Der schwalbenschwanzförmige Ansatz 17 am distalen Endabschnitt 14 des Schaftes 12 sorgt dann für einen exakt ausgerichteten und Relativdrehungen verhindernden Sitz.

Es ist auch möglich, den Schlitz 64 im Bereich des mittigen Abschnittes 70 etwas breiter auszuüben, so dass der Schaft 12 axial versetzt zunächst seitlich an die Schale 62 angelegt bzw. eingelegt wird und dann durch axiales Verschieben die durchmesservergrößerten Abschnitte 26 und 14 des Schaftes 12 axial in die entsprechenden durchmesservergrößerten Abschnitte 66 und 72 des Außenteils 60 eingeschoben werden. Dieser endgültige Sitz ist in Fig. 1 dargestellt, die Schiebebewegungen sind in Fig. 2 durch den Pfeil 75 symbolisiert.

Wie insbesondere aus den Darstellungen von Fig. 2 und 3 ersichtlich ist, ist die Mittellängsachse 67 des distalen Abschnittes 66 des Außenteils 60 seitlich gegenüber der Mittellängsachse 71 des mittigen Abschnittes 70 versetzt.

Gleiches gilt für den proximalen Abschnitt 72 der Schale 62 des Außenteils 60.

Der Versatz ist dabei derart, dass diese drei Abschnitte 66, 70 und 72 zumindest eine gemeinsame Mantellinie 76 aufweisen.

Dies erlaubt eine exakte Führung an einer zylindrischen Innenwand eines Trokares, über den das Endoskop 10 in einen Körper eingeschoben wird.

An der Außenseite der Schale 62 des Außenteils 60 ist ein Ablenkmechanismus 80 angeordnet.

Der Ablenkmechanismus 80 weist einen Hebel 82 in Form einer Lasche auf, der über einen Zapfen 84 verschwenkbar an der Außenseite des proximalen Abschnitts 72 der Schale 62 angeordnet ist. Im Abstand zu der Schwenkachse des Zapfens 34 ist ein Betätigungselement 86 in Form eines sich lang erstreckenden Drahtes 88 befestigt. Ein proximales Ende 90 des Drahtes 88 ist durch eine hier nicht näher dargestellte Öffnung im Hebel 82 durchgesteckt und unverlierbar an diesem gehalten.

Das distale Ende 92 des Drahtes 88 ist an der Außenseite des distalen Abschnittes 66 der Schale 62 befestigt.

Der Draht 88 ist ferner um einen Umlenkzapfen 94 geführt, der etwas im proximalen Abstand zu einem flexiblen Bereich 74 der ansonst steif ausgebildeten Schale 62 liegt. Der flexible Bereich 74 besteht in ziehharmonikaartigen Faltungen des Materials der Schale 62. Die Geometrie der Anlenkungspunkte von Zapfen 84 und Draht 88 ist nun so gewählt, dass, falls der Hebel aus der von Fig. 3 dargestellten Position in die in Fig. 4 dargestellte Position verschwenkt wird, wie das durch eine Pfeil 89 angedeutet wird, der distale Abschnitt 66 der Schale 62 gleichsinnig verschwenkt wird, wie das in Fig. 4 durch den Pfeil 91 angedeutet ist.

Diese Auswahl der Geometrie erleichtert die Handhabung dahingehend, dass der Operateur weiß, wenn er den Hebel 82 in diese Richtung verschwenkt auch der distale Abschnitt 66 des Außenteils in die gleiche Richtung verschwenkt wird.

Durch diese Verschwenkung wird auch der distale Endabschnitt 14 des Schaftes 12 gleichsinnig verschwenkt, denn dieser distale Endabschnitt 14 ist ja in dem distalen Abschnitt 66 des Außenteils 60 eingeschoben. Somit erfolgt damit auch zugleich und zwangsweise, aber passiv, die Ablenkung des distalen Endabschnittes 14 des Schaftes 12.

Dies wird auch dadurch möglich, dass der flexible Bereich 16 am Schaft 12 vorgesehen ist.

Die Hebelgeometrie ist auch so gewählt, dass, falls der Hebel 82 in die in Fig. 4 verschwenkte Position bewegt wurde, das System selbsthaltend ist, d.h. er nicht wieder von selbst in die in Fig. 3 dargestellte Position zurückkehrt.

Es kann die Geometrie auch so gewählt sein, dass der Hebel 82 aus der in Fig. 4 dargestellten Position wieder in die in Fig. 3 dargestellte Position zurückkehrt, wobei diese Rückstellung durch die Feder 20 gefördert wird, die um den flexiblen Bereich 16 des Schaftes 12 gelegt ist.

Hier kann man also auf die Wünsche der Handhabungsperson flexibel reagieren und entsprechende Ausgestaltungen bewerkstelligen.

Beim praktischen Einsatz kommt der Schaft 12 samt distalem Endabschnitt 14 und auch der entsprechende distale Abschnitt und Teile des mittigen Abschnittes 70 des Außenteiles 60 im Innern eines Trokars zum Liegen, durch den das Endoskop 10 bei einem Eingriff in einen Körper eingeführt wird.

Der Hebel 82 ragt proximalseitig von dem Trokar vor, damit die Handhabungsperson diesen betätigen kann.

Wenn das Endoskop in der in Fig. 4 dargestellten Position, also in der abgelenkten Stellung, nun um dessen Längsachse gedreht wird, dreht sich ja entsprechend das Bild 54 auf dem Monitor 52.

Falls erwünscht ist, dass das Bild 54 immer in einer gleichen Ausrichtung steht, beispielsweise, dass die Dreieckseite des hier beispielhaft dreieckseitigen Bildes 54, immer an der Unterkante des Monitors 52 zum Liegen kommt, kann dieses über die zuvor erwähnte Drehbarkeit des Bildsensors 42 über den Mikromotor 46 erreicht werden. Der Gravitations- oder Positionssensor 44 erlaubt die Aufrichtung des Bildsensors 42 in die entsprechend gewünschte, gleichbleibende Ausrichtung.

Dies kann über die Kontrolleinheit 50 steuerbar oder auch programmierbar, also auch automatisiert ablaufen, je nachdem wie das erwünscht ist. In Fig. 1 und 2 ist der Ablenkmechanismus 80 auf einer Seite an der Schale 62 des Außenteils 60 angeordnet.

Es ist auch vorgesehen, spiegelbildlich noch einen zweiten Ablenkmechanismus 80' vorzusehen, wie das in Fig. 3 durch den entsprechenden Hebel 82' angedeutet ist bzw. wie das aus der Ansicht von Fig. 7 ersichtlich ist.

Die Anlenkgeometrie des Ablenkmechanismus 80' kann beispielsweise so gewählt sein, dass mit diesem Ablenkmechanismus das Überbringen aus der abgelenkten Position von Fig. 4 in die geradlinige Ausrichtung von Fig. 3 bewerkstelligt werden kann.

Es kann auch vorgesehen sein, dass dieser Ablenkmechanismus 80' in der gleichen Richtung wirkt nur eine schon von vornherein andere ausgängliche Stellung des Hebels 82' beinhaltet, weil beispielsweise die Bedienung so gewünscht ist.

Wie insbesondere aus Fig. 5 ersichtlich ist, wird der Schaft 12 des Endoskops 10 durch die Feder 20, die um den flexiblen Bereich 16 gelegt ist, immer in dieser geradlinigen Ausrichtung gehalten, sofern keine äußeren Ablenkkräfte beispielsweise durch das Außenteil 60 einwirken. In dem dargestellten Ausführungsbeispiel ist nur der Schaft im Bereich des flexiblen Bereiches 16 flexibel, ansonsten ist er steif.

Somit ist der Schaft 12 bzw. das Endoskop 10 auch ohne angelegtes Außenteil 60 einzusetzen, nämlich als "klassisches" starres Endoskop, wenn das so gewünscht ist.

Ist der distale Endabschnitt 14 durch den Trokar hindurch in den Körper eingeschoben, ist neben dem durchmessergeringeren Abschnitt des Schaftes 12 ausreichend Platz im Trokar, um noch weitere Instrumente hin- und durchzuführen. Dies zeigt besonders die vielfältige Einsetzbarkeit des erfindungsgemäßen Endoskops 10.

## Patentansprüche

1. Endoskop mit einem Beleuchtungs- und Bildübertragungssystem aufnehmenden Schaft (12), wobei an den Schaft (12) ein Außenteil (60) angelegt ist, das einen Ablenkmechanismus (80, 80') zum Ablenken des Endoskopes (10) aufweist, wobei das Außenteil (60) zum Verbinden mit dem Schaft (12) seitlich an diesen anlegbar ist, wobei ein distaler Endabschnitt (14) des Schaftes (12) ablenkbar ausgebildet ist und der Übergang vom Schaft (12) zum distalen Endabschnitt (14) einen flexiblen Bereich (16) aufweist, **dadurch gekennzeichnet, dass** im flexiblen Bereich (16) des Schafts (12) ein Rückstellelement vorhanden ist, das kein Bauteil des Außenteils (60) ist, durch das Schaft (12) und starrer distaler Endabschnitt (14) in einen geradlinigen Ausrichtung verbleiben, und dass der Schaft (12) proximalseitig des flexiblen Bereiches (16), starr ausgebildet ist, und dass der Schaft (12) komplett aus metallischem Material hergestellt ist und wie bei einem starren metallischen Endoskop autoklavierbar ist.

2. Endoskop nach Anspruch 1, **dadurch gekennzeichnet, dass** das Rückstellelement (18) als ein federndes Element (20) ausgebildet ist.

3. Endoskop nach Ansprüch 1 oder 2, **dadurch gekennzeichnet, dass** der distale Endabschnitt (14) gegenüber dem Schaft (12) einen größeren Durchmesser (24) aufweist.

4. Endoskop nach einem der Ansprüche 1 bis 3, **dadurch gekennzeichnet, dass** der Schaft (12) proximalseitig einen Abschnitt (26) mit größerem Durchmesser (28) aufweist.

5. Endoskop nach einem der Ansprüche 1 bis 4, **dadurch gekennzeichnet, dass** das Außenteil (60) als zumindest ein teilweise seitlich offenes, schalenartiges Element (62) ausgebildet ist.

6. Endoskop nach Anspruch 5, **dadurch gekennzeichnet, dass** das Außenteil (60) eine seitliche Öffnung aufweist, die als Längsschlitz (64) ausgebildet ist, über den der Schaft (12) seitlich einclipsbar ist. 6

7. Endoskop nach einem der Ansprüche 1 bis 6, **dadurch gekennzeichnet, dass** proximalseitig und distalseitig am Außenteil (60) durchmessererweiterte Bereiche (66, 72) vorhanden sind, in die durchmessergrößere Bereiche (14, 26) des Schaftes (12) formschlüssig einbringbar sind.

8. Endoskop nach Anspruch 7, **dadurch gekennzeichnet, dass** die durchmessererweiterten Bereiche (14, 26) des Schaftes (12) axial in die durchmessererweiterten Bereiche (66, 72) des Außenteils (30) einschiebbar sind.

9. Endoskop nach einem der Ansprüche 3 bis 8, **dadurch gekennzeichnet, dass** eine Mittellängsachse (67) des distalen Abschnittes (66) des Außenteils (60) seitlich versetzt zur Mittellängsachse (71) eines mittigen Abschnittes (70) des Außenteils (60) verläuft.

10. Endoskop nach Anspruch 9, **dadurch gekennzeichnet, dass** der seitliche Versatz derart ist, dass mittiger Abschnitt (70) und distaler Endabschnitt (66) zumindest eine gemeinsame Mantellinie (76) aufweisen.

11. Endoskop nach einem der Ansprüche 1 bis 10, **dadurch gekennzeichnet, dass** der Ablenkmechanismus (80, 80') derart ausgestaltet ist, dass durch diesen ein distaler Abschnitt (66) des Außenteils (60) ablenkbar ist, in dem der distale ablenkbare Endabschnitt (14) des Schaftes (12) aufgenommen ist.

12. Endoskop nach Anspruch 11, **dadurch gekennzeichnet, dass** der Ablenkmechanismus (80, 80') ein sich von distal nach proximal erstreckendes Betätigungselement (86) aufweist, das distal mit dem ablenkbaren Abschnitt (66) des Außenteils (60) und proximalseitig mit einem Schwenkhebel (82) verbunden ist.

13. Endoskop nach einem der Ansprüche 1 bis 12, **dadurch gekennzeichnet, dass** das Außenteil (60) als Wegwerfteil ausgebildet ist.

14. Endoskop nach einem der Ansprüche 1 bis 13, **dadurch gekennzeichnet, dass** im ablenkbaren distalen Endabschnitt (14) des Schaftes (12) ein Bilderfassungssystem (41) des Bildübertragungssystems aufgenommen ist.

15. Endoskop nach Anspruch 14, **dadurch gekennzeichnet, dass** das Bilderfassungssystem (41) einen Bildsensor (42) mit Objektiv (40) aufweist.

16. Endoskop nach Anspruch 14 oder 15, **dadurch gekennzeichnet, dass** ein Sensor (44) vorhanden ist, der über Gravitationswirkung eine Position erkennbar macht.

17. Endoskop nach Anspruch 15 oder 16, **dadurch gekennzeichnet, dass** im distalen Endabschnitt (14) des Schaftes (12) ein Mikromotor (46) angeordnet ist, der mit dem Bildsensor (42) gekoppelt ist und diesen um dessen Bildsensorachse (43) dreht.

## Claims

1. Endoscope, with a shaft (12) that houses a lighting and an image transmission system, an outer part (60) is attached to the shaft (12), and which has a deflector mechanism (80, 80') for deflecting the endoscope (10), wherein for attaching the outer part (60) to the shaft (12) it can be mounted laterally thereon, a distal end portion (14) of the shaft (12) is deflectable, and a transition from the shaft (12) to the distal end portion (14) has a flexible area (16), **characterized in that** a resetting element is provided in the flexible area (16) of the shaft, which is not a component of the outer part (60), due to which the shaft (12) and the rigid distal end portion stay in the rectilinear orientation, and **in that** the shaft (12) is rigid in a portion proximally to the flexible area (16), and **in that** the shaft (12) is entirely made of a metallic material and is autoclavable like a rigid metallic endoscope.

2. Endoscope of claim 1, **characterized in that** the resetting element (18) is designed as a spring element (20).

3. Endoscope of claims 1 or 2, **characterized in that** the distal end portion (14) has a greater diameter (24) than the shaft (12).

4. Endoscope of any one of claims 1 through 3, **characterized in that** the shaft (12) has, at the proximal end, a portion (26) of greater diameter (28).

5. Endoscope of any one of claims 1 through 4, **characterized in that** the outer part (60) is designed as a shell-like element (62) at least partially open at the side.

6. Endoscope of claim 5, **characterized in that** the outer part (60) has a lateral opening designed as a longitudinal slit (64) via which the shaft (12) can be laterally clipped into place.

7. Endoscope of any one of claims 1 through 6, **characterized in that** areas (66, 72) of greater diameter are present at the proximal and distal ends of the outer part (60), and areas (14, 26) of greater diameter of the shaft (12) can be engaged with a form fit in shaft areas (66, 72).

8. Endoscope of claim 7, **characterized in that** the areas (14, 26) of greater diameter of the shaft (12) can be slid axially into the areas (66, 72) of greater diameter of the outer part (60).

9. Endoscope of any one of claims 3 through 8, **characterized in that** a central longitudinal axis (67) of the distal portion (66) of the outer part (60) is laterally offset in relation to the central longitudinal axis (71) of a central portion (70) of the outer part (60).

10. Endoscope of claim 9, **characterized in that** the lateral offset is such that the central portion (70) and the distal end portion (66) have at least a common surface (76).

11. Endoscope of any one of claims 1 through 10, **characterized in that** the deflector mechanism (80, 80') is designed in such a way that it can deflect a distal portion (66) of the outer part (60) in which the distal deflectable end portion (14) of the shaft (12) is received.

12. Endoscope of claim 11, **characterized in that** the deflector mechanism (80, 80') has an actuating element (86) that extends from distal to proximal and that is connected at the distal end to the deflectable portion (66) of the outer part and at the proximal end to a pivot lever (82).

13. Endoscope of any one of claims 1 through 12, **characterized in that** the outer part (60) is designed as a disposable part.

14. Endoscope of any one of claims 1 through 13, **characterized in that** an imaging system (41) of the imaging transmission system is housed in the deflectable distal end portion (14) of the shaft (12).

15. Endoscope of claim 14, **characterized in that** the imaging system (41) has an image sensor (42) with a lens (40).

16. Endoscope of claims 14 or 15, **characterized in that** a sensor (44) is present which, by the effect of gravitation, makes a position detectable.

17. Endoscope of claims 15 or 16, **characterized in that** a micromotor (46) arranged in the distal end portion (15) of the shaft (12) is coupled to the image sensor (42) and rotates this about its image sensor axis (43).

## Revendications

1. Endoscope comportant une tige (12) logeant un système d'éclairage et de transmission d'image, où est appliquée sur la tige (12) une partie externe (60) qui présente un mécanisme de déviation (80, 80') permettant de dévier l'endoscope (10), la partie externe (60) pouvant être disposée latéralement à la tige (12) pour la relier à celle-ci, un segment d'extrémité distal (14) de la tige (12) étant formé de façon à pouvoir être dévié et la transition entre la tige (12) et le segment d'extrémité distal (14) présentant une zone flexible (16), **caractérisé en ce que** dans la zone flexible (16) de la tige (12) se trouve un élément de rappel qui n'est pas une partie de la partie externe (60), par lequel la tige (12) et le segment d'extrémité distal rigide (14) restent dans une direction rectiligne, et **en ce que** la tige (12) côté proximal de la zone flexible (16) est exécutée de façon rigide, et **en ce que** la tige (12) est entièrement fabriquée dans un matériau métallique et peut être passée en autoclave comme dans le cas d'un endoscope métallique rigide.

2. Endoscope selon la revendication 1, **caractérisé en ce que** l'élément de rappel (18) est exécuté sous la forme d'un élément élastique (20).

3. Endoscope selon la revendication 1 ou 2, **caractérisé en ce que** le segment d'extrémité distal (14) présente un diamètre (24) supérieur à celui de la tige (12).

4. Endoscope selon l'une des revendications 1 à 3, **caractérisé en ce que** la tige (12) présente côté proximal un segment (26) présentant un diamètre supérieur (28).

5. Endoscope selon l'une des revendications 1 à 4, **caractérisé en ce que** la partie externe (60) est exécutée sous la forme d'au moins un élément (62) en forme de coque partiellement ouverte latéralement.

6. Endoscope selon la revendication 5, **caractérisé en ce que** la partie externe (60) présente une ouverture latérale qui est exécutée en tant que fente longitudinale (64) et via laquelle la tige (12) peut être enclipsée latéralement.

7. Endoscope selon l'une des revendications 1 à 6, **caractérisé en ce que** sont présentes au niveau de la partie externe (60) côté proximal et côté distal des zones de diamètre élargi (66, 72) dans lesquelles les zones de plus grand diamètre (14, 26) de la tige (12) peuvent s'engager par engagement de forme.

8. Endoscope selon la revendication 7, **caractérisé en ce que** les zones de diamètre élargi (14, 26) de la tige (12) peuvent être insérées axialement dans les zones de diamètre élargi (66, 72) de la partie externe (30).

9. Endoscope selon l'une des revendications 3 à 8, **caractérisé en ce que** l'axe longitudinal médian (67) du segment distal (66) de la partie externe (60) s'étend de façon décalée latéralement par rapport à l'axe longitudinal médian (71) d'un segment médian (70) de la partie externe (60).

10. Endoscope selon la revendication 9, **caractérisé en ce que** le décalage latéral est tel que le segment médian (70) et le segment d'extrémité distal (66) présentent au moins une ligne périphérique (76) commune.

11. Endoscope selon l'une des revendications 1 à 10, **caractérisé en ce que** le mécanisme de déviation (80, 80') est conçu de façon telle que par le biais de ce dernier, il est possible de dévier une extrémité distale (66) de la partie externe (60) dans laquelle est logé le segment d'extrémité distal pouvant être dévié (14) de la tige (12).

12. Endoscope selon la revendication 11, **caractérisé en ce que** le mécanisme de déviation (80, 80') présente un organe d'actionnement (86) qui s'étend du côté distal vers le côté proximal et qui est relié côté distal avec le segment pouvant être dévié (66) de la partie externe (60) et côté proximal avec un levier de basculement (82).

13. Endoscope selon l'une des revendications 1 à 12, **caractérisé en ce que** la partie externe (60) est conçue en tant que partie jetable.

14. Endoscope selon l'une des revendications 1 à 13, **caractérisé en ce que** dans le segment d'extrémité distal, pouvant être dévié, (14) de la tige (12) est logé un système de capture d'image (41) du système de transmission d'image.

15. Endoscope selon la revendication 14, **caractérisé en ce que** le système de capture d'image (41) présente un capteur d'image (42) avec un objectif (40).

16. Endoscope selon la revendication 14 ou 15, **caractérisé en ce qu'**il existe un capteur (44) qui permet d'identifier une position par l'effet de la gravitation.

17. Endoscope selon la revendication 15 ou 16, **caractérisé en ce que** dans le segment d'extrémité distal (14) de la tige (12) est disposé un micromoteur (46) qui est couplé au capteur d'image (42) et le fait tourner autour de son axe de capteur d'image (43).
